## Europäisches Patentamt

## European Patent Office

## Office européen des brevets

(19)

(11) Veröffentlichungsnummer: **0 141 865**
**B1**

(12)

# EUROPÄISCHE PATENTSCHRIFT

(45) Veröffentlichungstag der Patentschrift:
**18.05.88**

(51) Int. Cl.⁴: **A 61 N 1/42**

(21) Anmeldenummer: **83111002.8**

(22) Anmeldetag: **04.11.83**

(54) **Magnetfolien und Verfahren zu ihrer Befestigung.**

(43) Veröffentlichungstag der Anmeldung:
**22.05.85 Patentblatt 85/21**

(45) Bekanntmachung des Hinweises auf die Patenterteilung:
**18.05.88 Patentblatt 88/20**

(84) Benannte Vertragsstaaten:
**AT BE CH DE FR GB IT LI LU NL SE**

(56) Entgegenhaltungen:
**EP-A-0 076 354**
**EP-A-0 081 109**
**WO-A-82/00951**
**DE-A-2 362 908**
**DE-U-8 128 911**
**FR-A-1 429 342**
**FR-A-2 423 233**

(73) Patentinhaber: **LACOTHERM AG, Kronenstrasse 191,
CH- 9427 Wolfhalden (CH)**

(72) Erfinder: **Latzke, Arno Walter, Mühltobel 946, CH-
9429 Zelg/Wolfhalden (CH)**

(74) Vertreter: **Werner, Hans- Karsten, Dr.,
Deichmannhaus am Hauptbahnhof, D-5000 Köln 1
(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 141 865 B1

LIBER, STOCKHOLM 1988

## Beschreibung

Gegenstand der vorliegenden Erfindung sind Magnetfolien mit vorzugsweise streifenförmiger abwechselnder positiver und negativer Magnetisierung in Abständen von 1 bis 12 mm, vorzugsweise 3 bis 6 mm mit Feldstärken von 300 bis 1000 Gauss und ein Verfahren zur Befestigung derselben insbesondere an bewegten und/oder behaarten Körperteilen.

Es ist bekannt, die therapeutische Wirkung von Magnetfeldern einzusetzen mit Hilfe aufwendiger Apparaturen oder durch magnetische Heilpflaster, die aus einem Ferritplättchen und einem selbstklebenden Heftpflaster bestehen. Derartige magnetische Heilpflaster sind beispielsweise aus dem deutschen Gebrauchsmuster 79 19 808 bekannt. Der Nachteil dieser magnetischen Heilpflaster besteht darin, daß sie ihre Wirkung nur dann entfalten, wenn sie an den richtigen Stellen aufgeklebt werden. Das Auffinden der optimalen Befestigungspunkte ist insbesondere für den Laien, aber auch für den weniger routinierten Heilpraktiker oder Arzt mit Schwierigkeiten verbunden.

Aus dem deutschen Gebrauchsmuster 81 28 911 sind Magnetpflaster bekannt, welche aus einer 0,2 bis 2 mm starken, flexiblen, hautverträglichen, permanent magnetisierbaren und magnetisierten Kunststoff-Folie bestehen, welche entweder selbstklebend ist oder mit Hilfe von hautverträglichen, selbstklebendem Pflaster auf der Haut befestigt wird. Der Nachteil derartiger Magnetpflaster ist, daß die Behandlungsfläche noch immer relativ klein ist, da sich großflächige Pflaster nur unbequem tragen lassen und insbesondere an behaarten Körperteilen entweder schlecht haften oder sich nur sehr unangenehm wieder entfernen lassen. Aus dem deutschen Gebrauchsmuster 83 13 968 sind hautverträgliche Pflaster zur Befestigung von Magnetfolien bekannt, welche geeignet sind, Magnetfolien mehrfach zur Anwendung zu bringen. Aber auch diese Befestigung hat noch immer den Nachteil, daß sie an bewegten und/oder behaarten Körperteilen schlecht haftet oder schlecht wieder zu entfernen ist. Ein weiterer Nachteil aller bisher bekannten selbstklebenden oder aufgeklebten Magnetfolien ist, daß sie bei längerer und mehrfacher Verwendung brechen und dadurch unbrauchbar werden. Die Erfindung hat sich die Aufgabe gestellt, an sich bekannte Magnetfolien mit vorzugsweise streifenförmiger, abwechselnder positiver und negativer Magnetisierung in Abständen von 1 bis 12 mm, vorzugsweise 3 bis 6 mm mit Feldstärken von 300 bis 1000 Gauss so an Körperteilen, insbesondere an bewegten und/oder behaarten Körperteilen zu befestigen, daß sie gut haften, sich gut entfernen lassen, mehrfach anwendbar sind und eine längere Lebensdauer aufweisen. Diese Aufgabe wird erfindungsgemäß dadurch gelöst, daß man die Magnetfolien auf einer Seite mit Textilgewebe beklebt, welches ganz flächig oder in Teilbereichen mit einem Klettverschluß versehen ist und die so erhaltenen Magnetfolien mit elastischen Bändern mit Klettverschlüssen auf die Körperteile bindet.

Gegenstand der Erfindung sind somit Magnetfolien mit vorzugsweise streifenförmiger abwechselnder positiver und negativer Magnetisierung in Abständen von 1 bis 12 mm, vorzugsweise 6 mm mit Feldstärken von 300 bis 1000 Gauss, dadurch gekennzeichnet, daß eine Seite der Magnetfolie mit Textilgewebe beklebt ist, welches ganzflächig oder in Teilbereichen mit einem Klettverschluß versehen ist.

Das Textilgewebe kann in üblicherweise mit Textilklebern auf die Magnetfolien aufgeklebt werden. Der Klettverschluß kann auf dieses Textilgewebe zuvor aufgenäht oder aufgeklebt sein. Vorzugsweise wird die Magnetfolie mit einem Textilgewebe beklebt, welches als selbstklebendes Gewebe mit Klettverschluß vorliegt. Ein derartiges Material läßt sich besonders leicht und einfach auf der Magnetfolie aufbringen und kann gewünschtenfalls sogar nach einiger Zeit erneuert werden, sofern die Magnetfolie noch ausreichend gut erhalten ist. Ein besonderer Vorteil der neuen Befestigung von Magnetfolien besteht darin, daß zunächst einmal keinerlei Klebstoff auf die Haut oder den Körper gebracht werden muß, sodaß alle klebstoffbedingten Reizerscheinungen oder Allergien von vornherein ausgeschlossen werden können. Weiterhin ist es möglich, sowohl das Magnetpflaster als auch die elastischen Bänder mit Klettverschlüssen voneinander zu trennen und zu waschen oder zu reinigen. Dies macht die neue Befestigungsmethode besonders hygienisch und allergiefrei. Ein besonderer Vorteil der erfindungsgemäßen Magnetfolie ist, daß sie durch das aufgeklebte Textilgewebe mechanisch stabilisiert werden, ohne ihre Elastizität zu verlieren. Die bisher beobachtete Brüchigkeit der Folie bei längerem Gebrach wird erfindungsgemäß herabgesetzt oder ganz unterdrückt.

Von besonderer Bedeutung sind die neuen Magnetfolien und ihre Methode zur Befestigung in der Tiermedizin, wo insbesondere bei behaarten Tieren wie Pferden und Hunden selbstklebende Magnetfolien nicht zur Anwendung kommen können. Bei der Befestigung von Magnetfolien ohne Klebstoff mit Hilfe üblicher Bandagen, Bändern oder Klammern wurde bisher als sehr nachteilig empfunden, daß die Folien verrutschen oder sehr schnell brüchig werden und dadurch für eine Wiederverwendung kaum geeignet sind. Erfindungsgemäße Magnetfolien können daher mit besonderem Erfolg auch in der Tiermedizin eingesetzt werden. Darüberhinaus bietet sich ihre Anwendung an bei Sportverletzungen und der Behandlung bewegter und/oder behaarter Körperteile wie Gelenken, Armen, Beinen oder behaarten Kopfpartien.

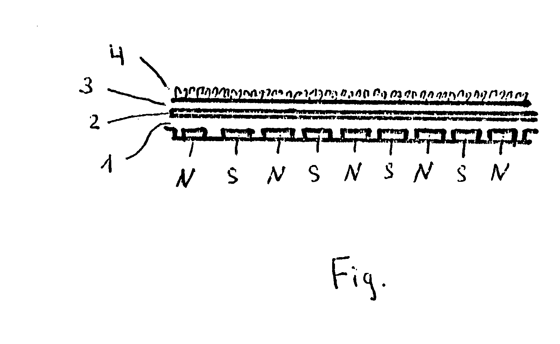

In der Fig. 1 ist ein Querschnitt durch eine bevorzugte Ausführungsform der erfindungsgemäßen Magnetfolien dargestellt.

Darin bedeuten:

1) eine streifenförmig abwechselnd positive und negativ magnetisierte Magnetfolien mit den Nordpolen (N) und Südpolen (S);
2) die Klebschicht;
3) das Textilgewebe;
4) der Klettverschluß.

Die erfindungsgemäßen Magnetfolien können als Folien oder Streifen oder sonstigen beliebigen Formen zugeschnitten sein, wobei die kleinste Abmessung im allgemeinen 2 cm und die größte etwa 40 cm ist. Vorzugsweise werden Abmessungen gewählt, die den bisher bekannten Abmessungen für Magnetfolien entsprechen, welche für die einzelnen Körperteile optimiert sein können.

In dem nachfolgenden Beispiel ist eine typische Ausführungsform der erfindungsgemäßen Magnetfolien näher erläutert.

**Beispiel**

Eine 1,5 mm starke flexible Magnetfolie, die streifenförmig abwechselnd magnetisiert ist und zwar jeweils 4 mm magnetisiert, 1 mm unverändert, danach 4 mm in der Gegenrichtung magnetisiert ist (lieferbar beispielsweise von der Firma Rheinmagnet Horst Baermann GmbH, Neunkirchen) wird auf die Dimension 5 x 8 cm zugeschnitten und auf der nicht-magnetisierten Seite mit einem selbstklebenden Textilband mit Klettverschluß beklebt. Die so erhaltene Magnetfolie kann in einfacher Weise mit elastischen Bändern mit Klettverschluß an den gewünschten Körperstellen befestigt werden, indem man sie mit der magnetisierten Seite zum Körper hin mit den Bändern festbindet. Die Magnetfolie rutscht nicht, liegt jedoch in der Weise am Körper an, daß sie ihre therapeutische Wirkung voll entfalten kann. Die Folie kann auf Wunsch jederzeit abgenommen und wieder aufgelegt werden. Sie kann leicht gereinigt werden. Die elastischen Bänder sind waschbar und können daher auch in sehr hygienischer Weise wieder verwendet werden. Selbstklebendes Textilband auf der Rückseite erhöht die Lebensdauer der Magnetfolie um ein Mehrfaches.

**Patentansprüche**

1. Magnetfolien mit vorzugsweise streifenförmiger abwechselnder positiver und negativer Magnetisierung in Abständen von 1 bis 12 mm, vorzugsweise 3 bis 6 mm mit Feldstärken von 300 bis 1000 Gauss, dadurch gekennzeichnet, daß eine Seite der Magnetfolie mit Textilgewebe beklebt ist, welches ganzflächig oder in Teilbereichen mit Klettverschluß versehen ist.

2. Magnetfolien gemäß Anspruch 1, dadurch gekennzeichnet, daß das Textilgewebe als selbstklebendes Gewebe mit Klettverschluß vorliegt.

3. Verfahren zur Befestigung von Magnetfolien mit vorzugsweise streifenförmiger abwechselnder positiver und negativer Magnetisierung in Abständen von 1 bis 12 mm, vorzugsweise 3 bis 6 mm mit Feldstärken von 300 bis 1000 Gauss insbesondere an bewegten und/oder behaarten Körperteilen, dadurch gekennzeichnet, daß man die Magnetfolien auf einer Seite mit Textilgewebe beklebt, welches ganz flächig oder in Teilbereichen mit einem Klettverschluß versehen ist und die so erhaltenen Magnetfolien mit elastischen Bändern mit Klettverschlüssen auf die Körperteile bindet.

**Claims**

1. A magnetic sheet having preferably an alternating stripe-shaped positive and negative magnetization at distances of from 1 to 12 mm, more preferably from 3 to 6 mm, with field strengths of from 300 to 1000 Gauss, characterized in that one side of the magnetic sheet is laminated with a textil fabric which is provided with a hook and loop-type (Velcro®) strip fastener on the entirety or only part(s) of its surface.

2. The magnetic sheet according to claim 1, characterized in that the textile fabric is in the form of a self-adhesive fabric with Velcro strip fastener.

3. A process for fixing a magnetic sheet having preferably an alternating stripe-shaped positive and negative magnetization at distances of from 1 to 12 mm, more preferably from 3 to 6 mm, with field strengths of from 300 to 1000 Gauss, preferably to parts in motion and/or hairy parts of the body, characterized in that the magnetic sheet is laminated on one surface thereof with a textile fabric which is provided with a hook and loop-type (Velcro®) strip fastener on the entirety or only part(s) of its surface and the magnetic sheet having been thus obtained is bound to the part of the body by means of elastic tapes having Velcro strip fasteners.

**Revendications**

1 . Lamelles magnétiques comportant de préférence une magnétisation par bandes alternativement positives et négatives à des écartements de 1 à 12 mm, et de préférence de 3 à 6 mm, avec des intensités de champ de 300 à 1000 gauss, caractérisées en ce qu'un côté de la lamelle magnétique est collé contre du tissu textile qui est garni, sur toute sa surface ou sur des régions partielles, d'un ruban de fixation à auto-accrochage, à boucles et crochets, adhérant par pression.

2. Lamelles magnétiques selon la revendication 1, caractérisées en ce que le tissu textile se présente comme un tissu auto-collant avec ruban de fixation à auto-accrochage, à boucles et crochets, adhérant par pression.

3. Procédé pour la fixation de lamelles magnétiques comportant de préférence une magnétisation par bandes alternativement positives et négatives à des écartements de 1 à 12 mm, et de préférence de 3 à 6 mm, avec des intensités de champ de 300 à 1000 gauss, en particulier sur des parties du corps mobiles et/ou couvertes de poils, caractérisé en ce qu'on colle les lamelles magnétiques, sur un côté, contre du tissu textile qui est garni, sur toute sa surface ou sur des régions partielles, d'un ruban de fixation à auto-accrochage, à boucles et crochets, adhérant par pression et on relie aux parties du corps les lamelles magnétiques ainsi obtenues par des bandes élastiques incluant de tels rubans de fixation.

Fig.

0 141 865